# EUROPÄISCHE PATENTANMELDUNG

(11) **EP 1 579 830 A1**
(43) Veröffentlichungstag der Anmeldung: **28.09.2005**
(21) Anmeldenummer: 05006320.5
(22) Anmeldetag: 22.03.2005
(51) Int. Cl.: A61F 7/00

(54) **Wärm- oder Kühlflasche mit Isolierschicht**

(30) Priorität: 22.03.2004 DE 102004014324; 27.08.2004 DE 102004041835
(71) Anmelder: Kraiss Martin, 89522 Heidenheim (DE)
(72) Erfinder: Kraiss Martin, 89522 Heidenheim (DE)

(57) **Zusammenfassung**

Bei der Erfindung handelt es sich um eine Wärmflasche für Menschen, oder Tiere. Diese dient dazu, die Benutzer der Wärmflasche zu wärmen, oder zu kühlen.

Die Wärmflasche ist von einer Isolierschicht 5 umgeben. Diese Isolierschicht ändert ihren Isolationswirkung mit der Änderung der Innentemperatur der Füllung 3 Wärmflasche.

Direkt nach dem Befüllen der Wärmflasche ist die Isolationswirkung am höchsten. Mit zunehmender Zeit nimmt die Isolationswirkung ab.

Die Isolationsschicht 5 verringert ihre Isolationswirkung indem mit zunehmender Zeit die gasförmige Füllung durch eine flüssige Füllung ersetzt wird.

## Beschreibung

Bei der Erfindung handelt es sich um eine Wärmflasche für Menschen, oder Tiere. Diese dient dazu, die Benutzer der Wärmflasche zu wärmen, oder zu kühlen.

Bekannte Wärmflaschen werden mit einer heißen Flüssigkeit, meistens Wasser, befüllt, oder haben eine Gelfüllung welche in der Mikrowelle erhitzt werden. Nach der Befüllung bzw. der Erhitzung wird ihnen keine Wärme mehr zugefügt. Die Wärmflaschen kühlen kontinuierlich ab, das heißt die Wärmemenge die nach außen abgegeben wird ist nicht konstant, sondern nimmt kontinuierlich ab. Anfangs geben sie sehr viel Wärmemenge nach außen ab. Zu dieser Zeit werden die Wärmflaschen für den Benutzer meist als zu heiß empfunden. Nach einer gewissen Zeit und nachdem sie schon einen Teil der in der Wärmflasche gespeicherten Wärmemenge abgegeben haben, geben die Wärmflaschen die ideale Wärmemenge nach außen ab. Diese empfindet der Benutzer als ideale Wärmemenge, bei der er sich wohlfühlt. Danach wird weiterhin Wärme nach außen abgegeben, jedoch zunehmend weniger. Diese geringere Abgabe an Wärme empfindet der Benutzer schließlich als nicht mehr wärmend genug.
Die Wärmflaschen können auch dazu benutzt werden, um zu kühlen. Diese nennt man dann auch Kühlflaschen. Dazu wird die mit einer Flüssigkeit oder Gel gefüllte Wärmflasche vor der Anwendung im Gefrierschrank heruntergekühlt, oder in sie wird kalte Flüssigkeit, oder Eiswürfel eingefüllt. Dabei tritt das gleiche Problem auf, wie bei der wärmenden Wärmflasche, nur in diesem Fall auf die Kälte bezogen. Die abgegebene Kaltemenge bleibt nicht konstant. Zu Anfang der Anwendung ist die Wärmflasche für den Benutzer zu kalt und später ist sie nicht mehr kühlend genug.

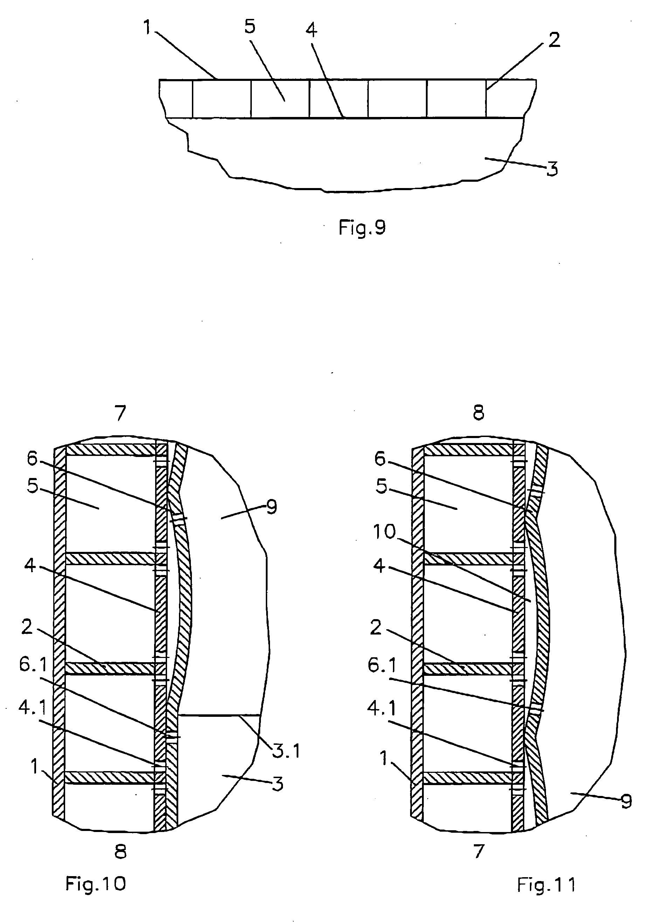

Die Erfindung löst das geschilderte Problem, indem die Wärmflasche von einer Isolierschicht umgeben ist. Diese Isolierschicht ändert ihren Isolationswirkung mit der Änderung der Innentemperatur der Wärmflasche. Die erfindungsgemäße Wärmflasche der Variante A besteht aus einer inneren Hülle 4 (Fig. 9), die mit heißem Wasser 3 gefüllt wird. Die innere Hülle 4 ist von einer äußeren Hülle 1 umgeben. Diese bildet mit der inneren Hülle 4 und Trennwänden 2 Hohlräume 5. Die äußere Hülle 1 und die Trennwände 2 sind wasserdicht. Die innere Hülle 4 ist wasserdurchlässig. Sie besteht aus einem sehr eng gewebten Gewebe, oder einer Folie mit sehr kleinen Löchern und lässt nur sehr wenig Wasser durch. Wird nun die Wärmflasche mit Wasser gefüllt, so sind die Hohlräume 5 zunächst komplett mit Luft gefüllt. Die Luft hat im Vergleich zu Wasser einen sehr niedrigen Wärmedurchgangskoeffizient, d.h. Luft isoliert mehr als Wasser. Die Isolierwirkung der Hohlräume 5 ist entsprechend höher, als wenn diese mit Wasser gefüllt sind. Mit der Zeit allerdings sickert das Wasser 3 mehr und mehr durch die innere Hülle 4. So füllt sich der Hohlraum 5 mit dem Wasser 3 aus der inneren Hülle 4. Damit nimmt die Isolierwirkung der Hohlräume, mit zunehmender Wasserfüllung, kontinuierlich ab. Dieser Vorgang vollzieht sich über mehrere Stunden. Am Schluss des Vorgangs sind die Hohlräume komplett mit Wasser gefüllt. Der Vorteil dabei ist, dass am Anfang des Vorgangs etwa die selbe Wärmemenge von der äußeren Hülle 1 nach außen abgegeben wird, wie nach ein paar Stunden, obwohl sich das Wasser 3 nach dieser Zeit schon deutlich abgekühlt hat (auf ca. 40°C). Dies kommt daher, dass am Anfang das sehr heiße Wasser (ca. 60-100° C) durch den luftgefüllten Hohlraum 5 sehr stark isoliert wird. Hingegen wird das abgekühlte Wasser 3 nach ein paar Stunden durch die wassergefüllten Hohlräume kaum noch isoliert und kann somit noch etwa die gleiche Wärmemenge an die äußere Hülle 1 abgeben, wie das heiße Wasser 3 zu Anfang des Abkühlvorgangs, Indem am Anfang des Vorgangs die von der Wärmflasche nach außen abgegebene Wärmemenge, durch die hohe Isolationswirkung, reduziert wird, wird die im Wasser 3 gespeicherte Wärmeenergie auch länger gespeichert. Die Wärmflasche wärmt somit länger, als herkömmliche Wärmflaschen, die schon zu Anfang des Abkühlvorgangs eine große Wärmemenge nach außen abgeben. Nach dem Benutzen der Wärmflasche wird der Wärmflaschenverschluss geöffnet und das Wasser 3, das sich innerhalb der inneren Hülle 4 befindet, fließt durch den geöffneten Wärmflaschenverschluss aus der Wärmflasche ab. Danach kann das Wasser, aus den Hohlräumen 5, zuerst in die innere Hülle 4 und anschließend durch den geöffneten Wärmflaschenverschluss aus der Wärmflasche abfließen. Alternativ kann die äußere Hülle 1 wasserdicht und gleichzeitig luftdurchlässig sein. In diesem Fall kann das in die Hohlräume 5 eingeströmte Wasser nicht durch die äußere Hülle 1 nach außen, d.h. außerhalb der Wärmflasche, gelangen. Jedoch kann die Luft aus den Hohlräumen 5 die äußere Hülle durchdringen und nach außen gelangen. Alternativ kann statt Wasser auch eine andere Flüssigkeit zum Füllen der inneren Hülle 4 verwendet werden (Beispielsweise Öl). In diesem Fall ist die innere Hülle 4 für diese Flüssigkeit durchlässig und die Trennwände 2 und äußere Hülle 1 für diese Flüssigkeit undurchlässig.

Eine weitere erfindungsgemäße Variante J der Wärmflasche entspricht von der Funktionsweise der Variante A. Allerdings befinden sich hier die Hohlräume 5 direkt zwischen der inneren Hülle 4 und der äußeren Hülle 1, die streckenweise miteinander verklebt oder verschweißt sind (Fig.8). Alternativ kann sich über der äußeren Hülle noch eine zweite äußere Hülle 1.1 (Fig.8 gestrichelt) befinden.

Eine weitere erfindungsgemäße Variante B der Wärmflasche entspricht vom Aufbau her den vorherig beschriebenen Varianten. Allerdings besteht die innere Hülle 4 aus einem Gewebe dessen Wasserdurchlässigkeit sich, in abängigkeit der Temperatur, erhöht oder senkt. Bei hohen Temperaturen (ca. 60-100° C) des Wassers nach dem Befüllen der Flasche, veringert sich die Wasserdurchlässigkeit des Gewebes. Bei zunehmender Abkühlung des Wassers 3 und somit auch des Gewebes, erhöht sich die Wasserdurchlässigkeit des Gewebes. Die Wasserdurchlässigkeit ist bei diesem Vorgang ist also nicht konstant, sondern nimmt mit zunehmender Abkühlung des Wassers 3 zu. Für die Hohlräume 5 bedeuted dies, dass sich die Hohlräume 5 nach dem Befüllen der Wärmflasche mit heißem Wasser 3 nur langsem mit dem Wasser 3 füllen. Kühlt das Wasser nach und nach ab, so erhöht sich die Wasserdurchlässigkeit des Gewebes und somit füllen sich die Hohlräume 5 auch schneller mit Wasser 3. Nach dem Benutzen der Wärmflasche wird das abgekühlte Wasser 3, das sich innerhalb der inneren Hülle 4 befindet, aus der Wärmflasche entleert und das Wasser aus den Hohlräumen 5 kann nun schnell durch die innere Hülle 4 abfließen, da die Wasserdurchlässigkeit der inneren Hülle 4 nun größer ist, als am Anfang direkt nach dem Befüllen der Wärmflasche mit heißem Wasser.

Eine weitere erfindungsgemäße Variante B1 der Wärmflasche entspricht vom Aufbau her der Variante B. Allerdings ist die Wärmflasche eine Kühlflasche. Ebenso wie in Variante B besteht die innere Hülle 4 aus einem Gewebe dessen Wasserdurchlässigkeit sich, in abängigkeit der Temperatur, erhöht oder senkt. Allerdings veringert sich in diesem Fall die Wasserdurchlässigkeit des Gewebes bei niedrigen Temperaturen (ca. 0-10° C) des Wassers nach dem Befüllen der Flasche. Bei zunehmender Erwärmung des Wassers 3 und somit auch des Gewebes, erhöht sich die Wasserdurchlässigkeit des Gewebes. Die Wasserdurchlässigkeit ist bei diesem Vorgang ist also nicht konstant, sondern nimmt mit zunehmender Erwärmung des Wassers 3 zu. Für die Hohlräume 5 bedeuted dies, dass sich die Hohlräume 5 nach dem Befüllen der Kühlflasche mit kaltem Wasser 3 oder Eis nur langsem mit dem Wasser 3 füllen. Erwärmt sich das Wasser 3 nach und nach, so erhöht sich die Wasserdurchlässigkeit des Gewebes und somit füllen sich die Hohlräume 5 auch schneller mit Wasser 3. Nach dem Benutzen der Kühlflasche wird das erwärmte Wasser 3, das sich innerhalb der inneren Hülle 4 befindet, aus der Wärmflasche entleert und das Wasser aus den Hohlräumen 5 kann nun schnell durch die innere Hülle 4 abfließen, da die Wasserdurchlässigkeit der inneren Hülle 4 nun größer ist, als am Anfang direkt nach dem Befüllen der Kühlflasche mit kaltem Wasser.

Eine weitere erfindungsgemäße Variante B2 der Wärmflasche entspricht vom Aufbau her der Variante B. Allerdings besteht die innere Hülle 4 aus einem Gewebe dessen Fasern einen hohen Wärmeausdehnungskoeffizient haben. Die hohen Temperaturen (ca. 60-100° C) des Wassers nach dem Befüllen der Flasche, erwärmen die Fasern stark. Diese dehnen sich im Volumen aus und verkleinern so die Zwischenräume zwischen den Fasern. In Folge sinkt die Wasserdurchlässigkeit des Gewebes. Bei zunehmender Abkühlung des Wassers und somit auch des Gewebes, schrumpfen auch wieder die Fasern, die Faserzwischenräume vergrößern sich und erhöhen somit die Wasserdurchlässigkeit des Gewebes. Die Wasserdurchlässigkeit ist bei diesem Vorgang nicht konstant, sondern nimmt mit zunehmender Abkühlung des Wassers 3 zu. Nach dem Benutzen der Wärmflasche wird das abgekühlte Wasser 3, das sich innerhalb der inneren Hülle 4 befindet, aus der Wärmflasche entleert und das Wasser aus den Hohlräumen 5 kann nun schnell durch die innere Hülle 4 abfließen, da die Wasserdurchlässigkeit der inneren Hülle 4 nun größer ist, als am Anfang direkt nach dem Befüllen der Wärmflasche mit heißem Wasser. Statt einem Gewebe kann die innere Hülle 4 auch aus einer Folie mit vielen kleinen Löchern und mit einem hohem Wärmeausdehnungskoeffizienten bestehen. In diesem Fall verschließen sich die Löcher bei zunehmender Erwärmung der Folie.

Eine weitere erfindungsgemäße Variante B3 besteht aus einer äußeren wasserdichten Hülle 1 und einem wasserdurchlässigen Schaumstoff 24 (Fig.60). Der Schaumstoff ist porös und nimmt das Wasser 3 nur sehr langsam auf. Bis der Schaumstoff vollständig mit Wasser gefüllt ist vergehen einige Minuten bis mehrere Stunden. Je mehr sich der Schaumstoff mit Wasser füllt, desto geringer wird seine Isolationswirkung. Je nachdem wie porös und in welcher Dicke der Schaumstoff 24 gewählt wird, variiert seine Isolationswirkung und die Zeit bis er vollständig mit Wasser 3 gefüllt ist.
Je nach Schaumstoffeigenschaften kann der Schaumstoff, während er sich mit Wasser 3 füllt, pro Zeiteinheit gleichmäßig viel Wasser 3 aufnehmen, oder er verändert seine Wasseraufnahmefähigkeit pro Zeiteinheit mit der sich ändernden Temperatur des Schaumstoffes. Die Temperatur des Schaumstoffes 24 ändert sich mit der Temperatur des Wassers 3, da das Wasser 3 an den Schaumstoff 24 angrenzt und in den Schaumstoff 24 einfließt. Hat der Schaumstoff 24 eine, mit einer Temperaturänderung einhergehende, veränderliche Wasseraufnahmefähigkeit, so ist dafür beispielsweise der sehr hohe Wärmeausdehnungskoeffizient des Schaumstoffes 24 verantwortlich, der bei einer Temperaturerhöhung die Poren des Schaumstoffes zunehmend verschließt und bei einer Temperaturabnahme des Schaumstoffes 24 die Poren zunehmend öffnet.

Eine weitere erfindungsgemäße Variante B4 der Wärmflasche entspricht vom Aufbau her den Varianten A, J, B, B1 und B2. Allerdings sind die Hohlräume 5 mit einem Schaumstoff gefüllt. Die Eigenschaften des Schaumstoffes entsprechen denen des Schaumstoffes der Variante B3.

Ein Problem der genannten erfindungsgemäßen Varianten A, J, B, B1, B2, B3 und B4 kann sein, dass sich Kalk im Gewebe oder den Löchern der inneren Hülle 4 ablagert und somit die Wasserdurchlässigkeit verringert. Das wird verhindert, wenn die Wärmflasche nur mit destilliertem Wasser gefüllt wird.

Eine weitere erfindungsgemäße Variante C der Wärm- oder Kühlflasche besteht aus einer inneren Hülle 4 und einer zweiten inneren Hülle 6. Die innere Hülle 4 ist von einer äußeren Hülle 1 umgeben. Diese bildet mit der inneren Hülle 4 und Trennwänden 2 Hohlräume 5. Die äußere Hülle 1 und die Trennwände 2 sind wasserdicht. Die innere Hülle 4 ist wasserdicht, hat aber pro Hohlraum 5 mindestens ein Loch 4.1 von wenigen Millimetern Durchmesser. Die zweite innere Hülle 6 ist wasserdurchlässig und besteht aus einem sehr eng gewebten Gewebe, oder einer Folie mit sehr vielen kleinen Löchern von weniger als einem Zehntel Millimeter die einen Abstand von 0,5 mm zueinander haben und lässt nur sehr wenig Wasser durch. Sie ist an der inneren Hülle 4 befestigt und besitzt mindestens ein Loch 6.1 mit mehr als einem Millimeter Durchmesser.
Fig. 10 zeigt einen Ausschnitt der Wärmflasche beim Befüllen der Wärmflasche mit Wasser 3. Dabei zeigt in dieser Darstellung die Richtung 7 nach oben zum Wärmflaschenverschluss durch den das Wasser von außen eingefüllt wird. Beim Befüllen steigt der Wasserspiegel 3.1 an und drückt dadurch die zweite innere Hülle 6 an die innere Hülle 4 an. Das Wasser fließt nur im Bereich Löcher 4.1 durch die zweite innere Hülle 6. Indem die innere Hülle 4 und die zweite innere Hülle 6 eng aneinander anliegen sind die Löcher 6.1 der zweiten inneren Hülle 6 verschlossen und das Wasser kann nur im Bereich der Löcher 4.1 die wasserdurchlässige zweite innere Hülle 6 durchdringen. Dies bedeutet das die Fläche der zweiten inneren Hülle 6, die vom Wasser durchdrungen wird sehr klein ist. Sie entspricht der Summe der addierten Flächen der Löcher 4.1, der inneren Hülle 4, die unter dem Wasserspiegel liegen, Entsprechend langsam füllen sie die Hohlräume 5 mit Wasser.
Zum Entleeren der Wärmflasche wird der Wärmflaschenverschluss geöffnet und die Wärmflasche gedreht, so dass der Verschluss nach unten zeigt. Dies ist in Fig. 11 dargestellt. 7 zeigt hier nach unten in Richtung zum Wärmflaschenverschluss. Sofort nachdem das Wasser 3 aus der zweiten inneren Hülle 6 abgeflossen ist, strömt das Wasser aus den Hohlräumen 5 durch die Löcher 4.1 der inneren Hülle 4 und drückt die zweite innere Hülle 6 von der inneren Hülle 4 weg. Das Wasser flutet den entstandenen Hohlraum 10 zwischen inneren Hülle 4 und der zweiten inneren Hülle 6 und fließt ungehindert durch die Löcher 6.1 der zweiten inneren Hülle 6 ab. Da das Wasser aus den Hohlräumen 5 komplett durch die Löcher 4.1 und 6.1 abfließen kann und nicht die zweite innere Hülle 6 an den Flächen der Löcher 4.1 der inneren Hülle 4 durchdringen muss, vollzieht sich das Abfließen viel schneller, als das Füllen der Hohlräume 5, nach dem Befüllen der Wärmflasche. Das Abfließen wird unter einer Minute dauern, das Füllen der Hohlräume 5 dagegen mehrere Stunden.
Alternativ ist die innere Hülle 4 wasserdurchlässig und hat die gleichen Löcher 4.1. In dieser Ausführung vollzieht sich das Füllen Hohlräume 5 mit Wasser schneller, aber dennoch langsamer als das Entleeren des Wassers.

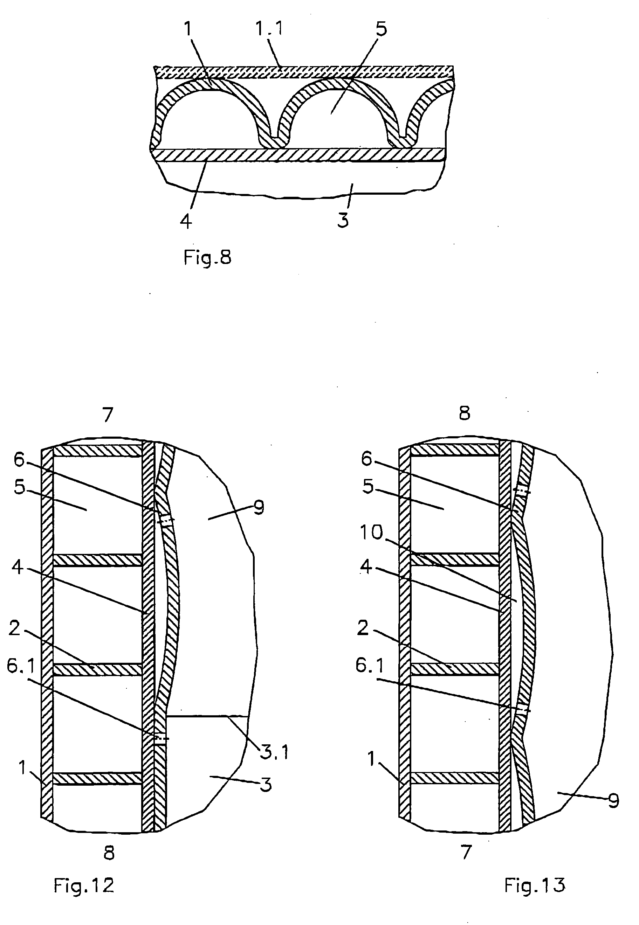

Eine weitere erfindungsgemäße Variante D der Wärm- oder Kühlflasche entspricht vom Aufbau her der vorhergehenden Variante C, allerdings fehlen die Löcher 4.1 der inneren Hülle 4 und diese ist wasserdurchlässig (Fig. 12 und 13). Die zweite innere Hülle 6 ist hingegen eine wasserdichte Folie, oder Gewebe, das die gleichen Löcher 6.1 wie Variante C besitzt. Nach dem Füllen der Wärmflasche füllen sich die Hohlräume 5 ebenso langsam wie bei der vorherigen Variante, allerdings leeren sich die Hohlräume 5 beim Entleeren der Wärmflasche langsamer, da keine Löcher in der inneren Hülle 4 vorhanden sind, jedoch leeren sich die Hohlräume 5 immer noch schneller, als sie sich nach dem Befüllen der Wärmflasche füllen, da beim Entleeren das Wasser der Hohlräume 5 die ganze Fläche der wasserdurchlässigen inneren Hülle 4 durchströmt und in den Hohlraum 10 strömen kann, von wo es endgültig durch die Löcher 6.1 der zweiten inneren Hülle 6 und anschließend durch den Wärmflaschenverschluss abfliesen kann.
Eine weitere erfindungsgemäße Variante entspricht vom Aufbau her der vorherigen Variante D, jedoch besitzt hier die innere Hülle 4 die Löcher 4.1 und ist aus wasserdichtem Material und die zweite innere Hülle 6 besitzt keine Löcher und ist wasserdurchlässig.

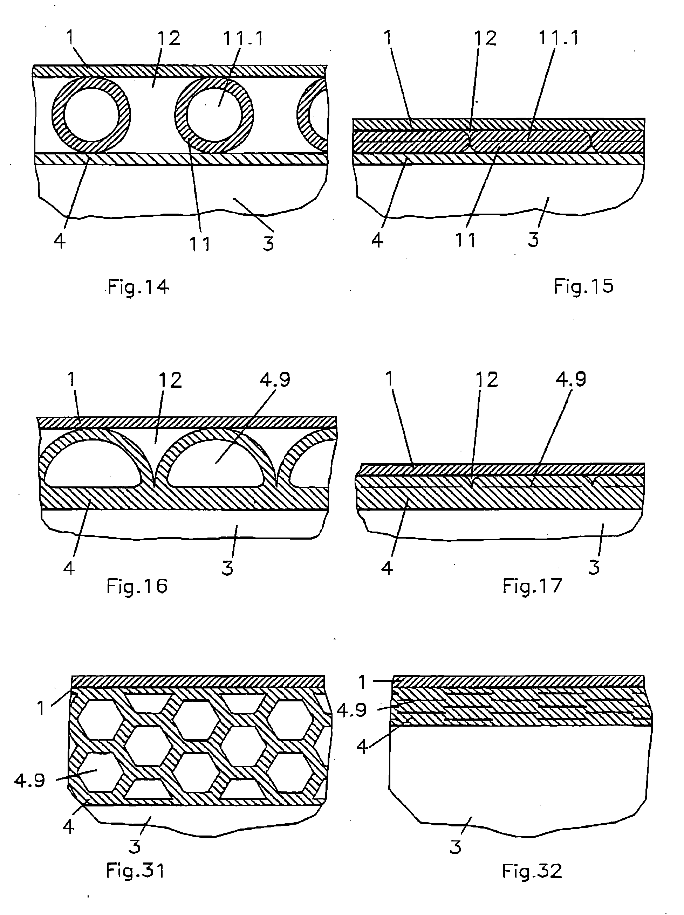

Eine weitere erfindungsgemäße Variante E der Wärmflasche besitzt eine äußere Hülle 1, eine innere Hülle 4 und zwischen den beiden Hüllen 1 und 4 befinden sich geschlossene Hohlkörper 11 (Fig.14). Die innere Hülle 4 und die Hohlkörper 11 sind wasserdicht. Der Hohlraum 11.1 der Hohlkörper 11 ist mit einem Medium gefüllt, das bei Raumtemperatur von etwa 20°C flüssig ist und bei einer Temperatur die höher liegt als die Siedetemperatur des Mediums (vorzugsweise zwischen 40° C und 80°C) gasförmig wird. Alkohol hat beispielweise diese genannten Eigenschaften. Methylalkohol hat eine Siedetemperatur von 66°C. Ethanol mit seiner Siedetemperatur von 78°C eignet sich insbesondere gut, weil es ungiftig ist und bei einem Austreten aus dem Hohlkörper keinen Schaden an Mensch und Umwelt anrichten kann. Wird die innere Hülle 4 der Wärmflasche mit einer Flüssigkeit, vorzugsweise Wasser 3, gefüllt, die wärmer ist, als die Siedetemperatur des Mediums, dann wird das Medium im Hohlraum 11.1 ebenfalls erwärmt und wird, sobald es seine Siedetemperatur erreicht hat, gasförmig, dehnt sich somit aus und bläht den Hohlkörper 11 zu einer kreisrunden Form auf (Fig. 14). Dadurch entsteht zwischen der äußeren Hülle 1 und der inneren Hülle 4 ein Hohlraum 12, der sich mit Luft füllt. Die Luft strömt dabei durch die äußeren Hülle 1 von außen ein. Durch die Luft im Hohlraum 12 entsteht eine Isolierung zwischen der äußeren Hülle 1 und der inneren Hülle 4. Kühlt sich das Wasser 3 in der inneren Hülle 4 ab, so kühlt auch das Medium im Hohlraum 11.1 des Hohlkörpers 11 ab. Sobald nun die Temperatur des Mediums unter seinen Siedepunkt bzw. den Dampfpunkt fällt, so kondensiert es, wird flüssig, zieht somit den Hohlkörper 11 zusammen und leert den Hohlraum 12 zwischen der äußeren Hülle 1 und der inneren Hülle 4 (Fig.15). Indem der luftgefüllte Hohlraum 12 somit nicht mehr besteht, hat er auch keine isolierende Wirkung mehr.

Alternativ kann die innere Hülle 4 statt mit einer Flüssigkeit auch mit einem Gel, oder mit Granulat, oder mit Körnern gefüllt sein. In diesem Falle wird die Wärmflasche in der Mikrowelle oder im Ofen erhitzt.

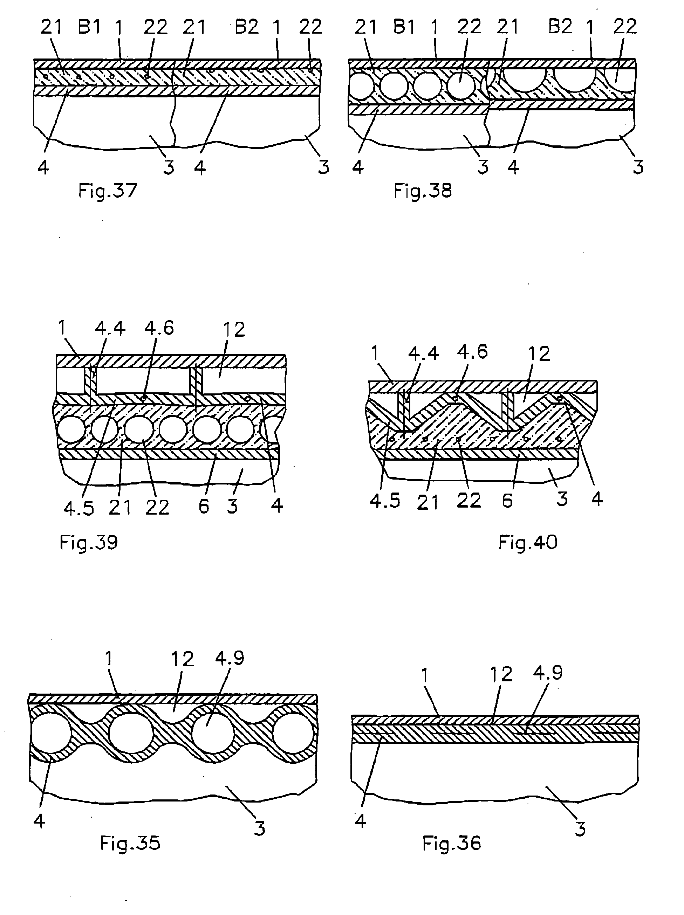

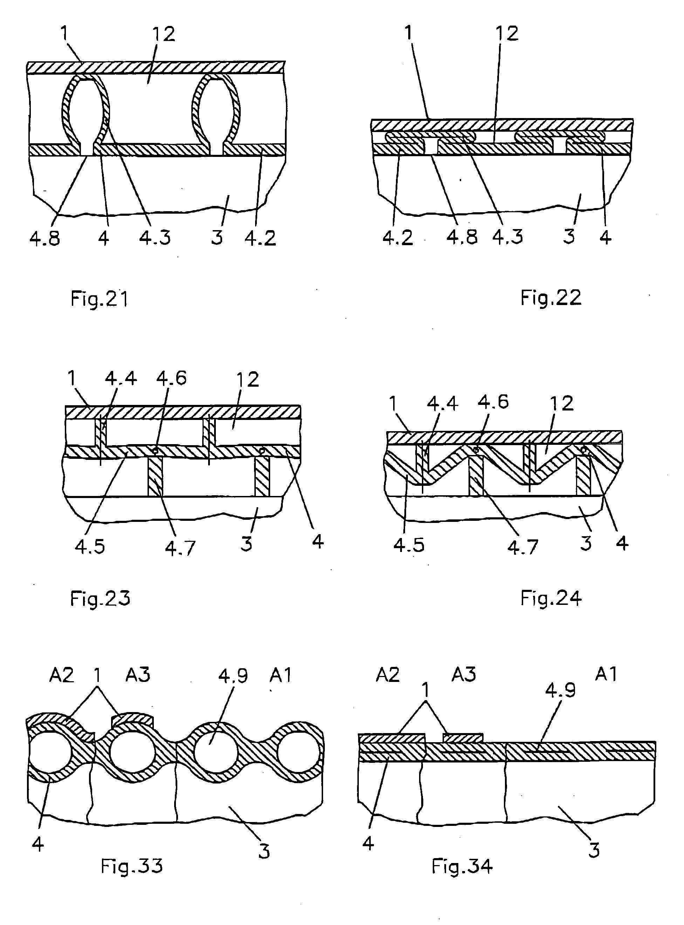

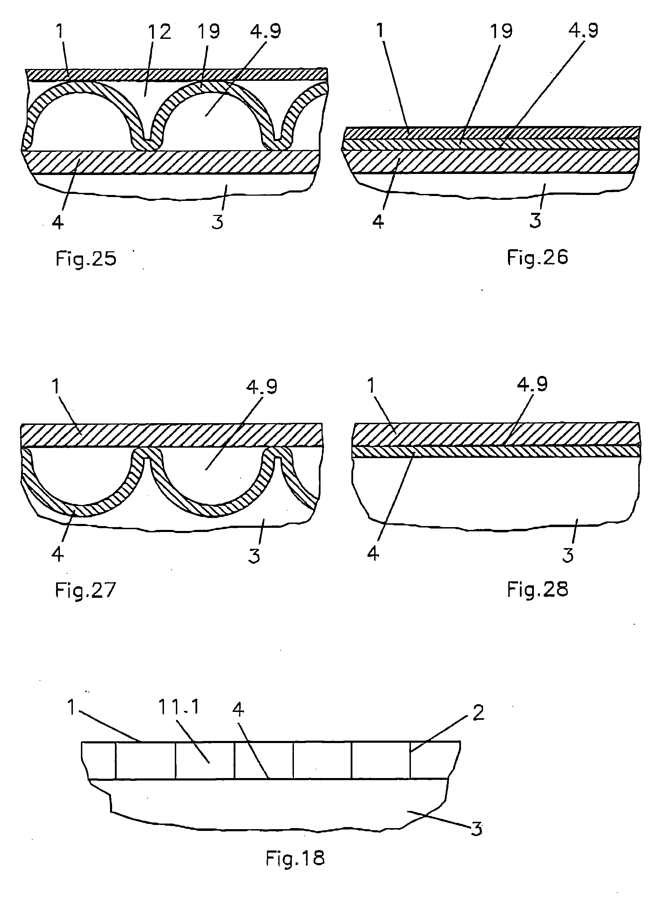

Eine weitere erfindungsgemäße Variante F der Wärmflasche funktioniert nach dem gleichen Funktionsprinzip wie die Variante E. Allerdings sind hier die mit dem Medium gefüllten Hohlräume 4.9 in der inneren Hülle 4 integriert. Fig. 16 zeigt die Variante mit dampfförmigem Medium und in Fig. 17 mit flüssigem Medium. Die gleiche Variante jedoch mit kreisrunden Hohlräumen 4.9 zeigen Fig. 35 und 36 (Medium dampfförmig Fig.35, Medium flüssig Fig.36).

Ebenso funktioniert die Variante F1 und F2 jedoch gibt es keinen luftgefüllten Hohlraum 12.
In Variante F1 sind die Hohlräume 4.9 übereinander angeordnet. In Fig.31 ist das Medium dampfförmig und in Fig.32 flüssig.
In Variante F2 sind die Hohlräume 4.9 nebeneinander angeordnet. In Fig.33 ist das Medium dampfförmig und in Fig.34 flüssig. Alternativ gibt es keine äußere Hülle 1 A1, oder die innere Hülle 4 ist von einer isolierenden äußeren Hülle 1 ganz A2, oder teilweise A3 überzogen.

Eine weitere erfindungsgemäße Variante G der Wärmflasche funktioniert nach dem gleichen Funktionsprinzip wie die Variante E. Allerdings werden hier die Hohlräume durch eine Folie 19 gebildet, die auf die innere Hülle 4 geschweißt oder geklebt ist. In Fig.25 ist das Medium dampfförmig und in Fig.26 flüssig.

Eine weitere erfindungsgemäße Variante H der Wärmflasche funktioniert nach dem gleichen Funktionsprinzip wie die Variante E. Allerdings sind hier die Hohlräume 11.1 nicht durch einen runden Hohlkörper gebildet, sondern durch Trennwände 2 die die innere Hülle 4 und die äußere Hülle 1 miteinander verbinden. Der Hohlraum 12 entfällt. Fig. 18 zeigt das Medium in dampfförmigem Zustand.

Eine weitere erfindungsgemäße Variante I der Wärmflasche funktioniert nach dem gleichen Funktionsprinzip wie die Variante E. Allerdings befinden sich hier die mit dem Medium gefüllten Hohlräume 4.9 direkt zwischen der inneren Hülle 4 und der äußeren Hülle 1. Die innere 4 und die äußere Hülle 1 sind streckenweise miteinander verklebt oder verschweißt, so dass sie mehrere von sich abgetrennte Hohlräume 4.9 bilden. Fig. 27 zeigt das Medium in dampfförmigem und Fig. 28 zeigt das Medium in flüssigem Zustand.

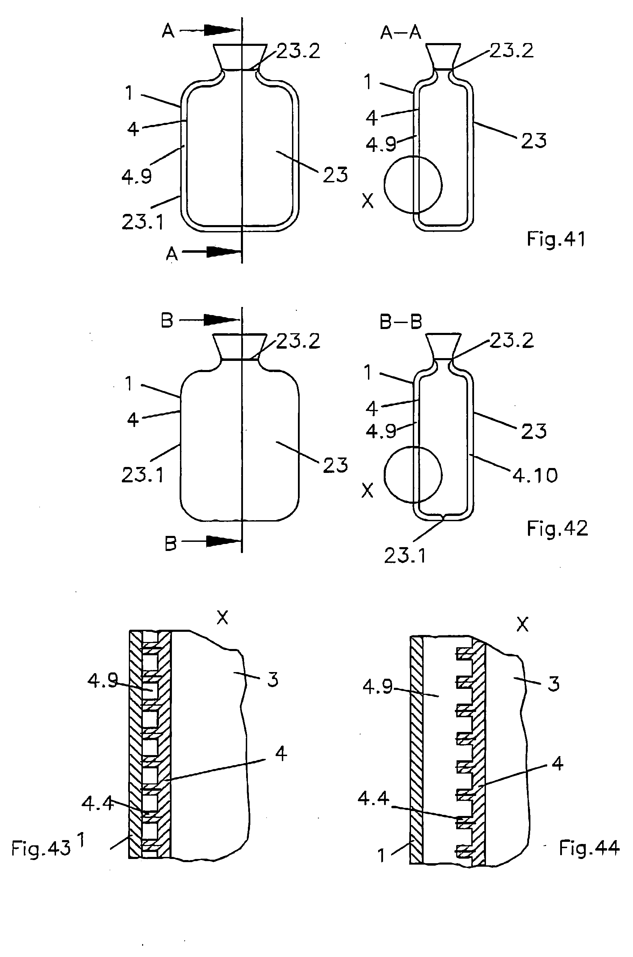

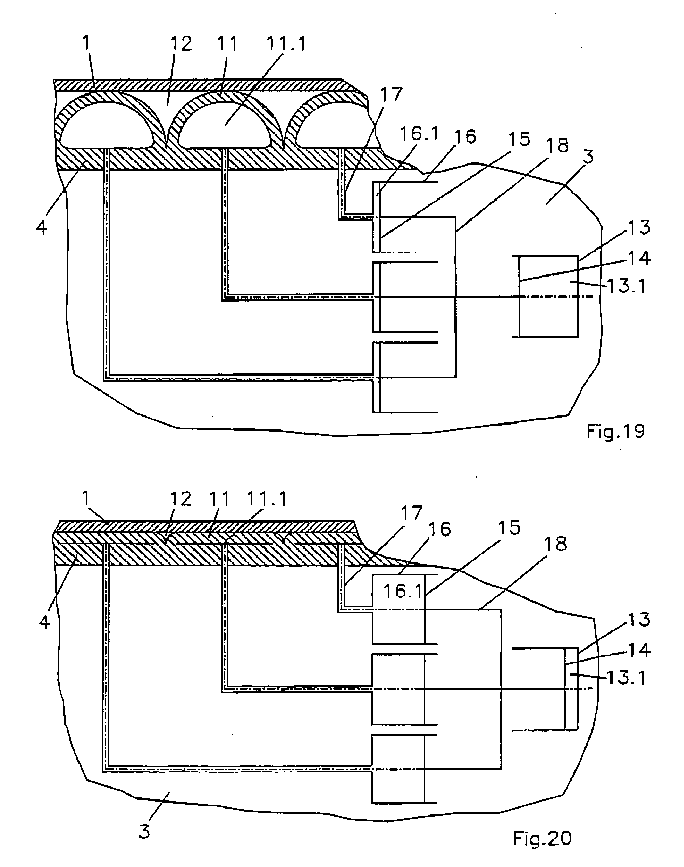

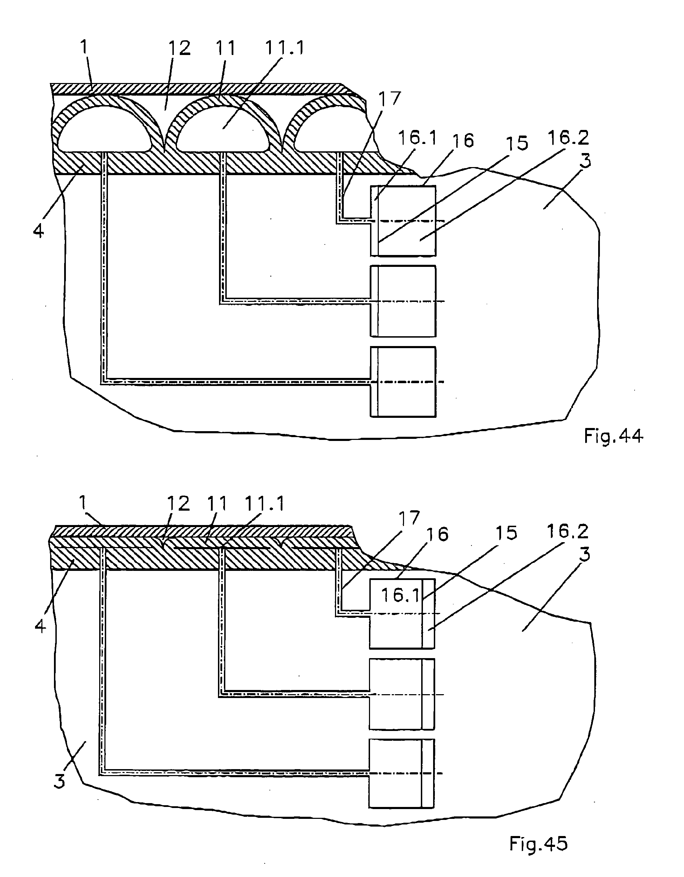

Eine weitere erfindungsgemäße Variante Z der Wärmflasche funktioniert nach dem gleichen Funktionsprinzip wie die Variante E. Allerdings ist hier die äußere Hülle 1 und die innere Hülle 4 nur im Bereich des Einfüllstutzens 23.2 der Wärmflasche 23 miteinander verklebt (Fig.41). So bildet sich zwischen der äußeren Hülle 1 und der inneren Hülle 2 ein Hohlraum 4.9 der mit dem Medium gefüllt ist. Zusätzlich können sie auch am Rand 23.1 der Wärmflasche 23 miteinander verklebt sein (Fig.42). In diesem Fall befinden sich zwischen der äußeren Hülle 1 und der inneren Hülle 4 zwei Hohlräume 4.9 und 4.10. Fig.43 und Fig. 44 zeigen einen vergrößerten Ausschnitt X der Fig.41 und Fig.42. Fig. 43 zeigt dabei das Medium in flüssigem Zustand und Fig.44 zeigt das Medium in dampfförmigem Zustand. Abstandshalter 4.4 bilden einen mit dem Medium gefüllten Hohlraum 4.9 zwischen der inneren 4 und der äußeren Hülle 1. Die Abstandshalter 4.4 sind Teil der inneren Hülle 4 (Fig.43 u. Fig.44) oder der äußeren Hülle 1 (ohne Darstellung). Befindet sich das Medium in flüssigern Zustand (Fig.43), so ist der Hohlraum 4.9 vollständig mit dem flüssigem Medium ausgefüllt und die Isolierwirkung ist geringer, als wenn sich das Medium in dampfförmigem Zustand befindet (Fig. 44).

Eine weitere erfindungsgemäße Variante K der Wärmflasche besitzt wie die Varianten E, F, G, Hohlräume 11.1 die sich außerhalb der inneren Hülle 4 befinden oder Hohlräume 4.9 in die innere Hülle 4 integriert sind. Jeder Hohlraum ist über eine Leitung mit einem Reservoir verbunden, oder das Reservoir schließt direkt an den Hohlraum an und ist über eine Öffnung mit dem Hohlraum räumlich verbunden, d.h. das Reservoir bildet mit dem Hohlraum einen gemeinsamen geschlossenen Hohlraum. Indem das Reservoir aus einem steifen Material besteht, beispielsweise aus Hartplastik, hat es bei allen Betriebstemperaturen ein nahezu konstantes Volumen. Die Außenwand des Reservoirs hat Kontakt zu dem Wasser 3, mit dem die innere Hülle 4 der Wärmflasche gefüllt ist. Bei normaler Raumtemperatur von etwa 20°C ist das Reservoir voll mit Wasser gefüllt und die Hohlräume 11.1 bzw. 4.9 sind leer und zusammengezogen. Erhöht sich die Temperatur, indem die Wärmflasche, bzw. die innere Hülle 4, mit heißem Wasser 3 gefüllt wird, so erwärmt sich auch das Wasser im Reservoir, dehnt sich aus und vergrößert somit sein Volumen. Da das Reservoir ein nahezu konstantes Volumen hat und sich das Volumen des Wassers bei Erwärmung stärker vergrößert, als das Volumen des Reservoirs, strömt das überschüssige Wasser in die Hohlräume 11.1 bzw. 4.9 und füllt diese. Infolgedessen bilden sich Hohlräume 12, indem Luft durch die äußeren Hülle 1 von außen einströmt. Durch die Luft im Hohlraum 12 entsteht eine Isolierung zwischen der äußeren Hülle 1 und der inneren Hülle 4.

Eine weitere erfindungsgemäße Variante L der Wärmflasche besitzt die gleichen Hohlräume 11.1 bzw. 4.9 wie die Variante E, F, G, H, I und einen Zylinder 13 mit einem Kolben 14 (Fig.19 und 20). Dieser Kolben 14 ist über Hebel, oder Stangen 18 mit weiteren Kolben 15 in Zylindern 16 verbunden. Jeder dieser Zylinder 16 ist über eine Leitung 17 mit je einem Hohlraum 11.1 verbunden. Der Hohlraum 16.1 auf der Kolbenseite des Zylinders 16 ist mit Luft oder Gas gefüllt. Sind die Hohlräume wie in den Varianten E, F, G, gestaltet, so eignet sich auch Flüssigkeiten, wie Wasser oder Öl als Füllung. Der Hohlraum 13.1 auf der einen Seite des Kolbens 14 ist mit einem Medium gefüllt, das bei normaler Raumtemperatur von etwa 20°C flüssig ist und dessen Siedepunkt zwischen 30°C und 100°C, vorzugsweise zwischen 35° C und 80°C, liegt. Diese Seite des Zylinders 13 ist nach außen hin geschlossen, somit kann das Medium auch nicht austreten. Dies ist insbesondere von Vorteil, wenn es sich bei dem Medium um ein giftiges handelt, beispielsweise um Äthyläther mit einem Siedepunkt von 35°C, oder Aceton mit 56°C, oder Petroläther mit >40°C. Die Außenwand des Zylinders 13 hat Kontakt zu dem Wasser 3, das in die innere Hülle 4 gefüllt wird. Wird heißes Wasser 3 in die innere Hülle 4 der Wärmflasche eingefüllt, so erhitzt sich auch das Medium im Hohlraum 13.1 des Zylinders 13. Erhitzt nun das heiße Wassers 3 das Medium im Zylinder 13 Ober dessen Siedepunkt, so verdampft das Medium, dehnt sich aus und drückt den Kolben 14 und über die Stangen 18 somit auch die Kolben 15 nach links. Somit verkleinert sich der Hohlraum 16.1 des Zylinders 16. Folglich wird die Luft des Hohlraums 16.1 über die Leitungen 17 in die Hohlräume 11.1 gedrückt und diese werden aufgeblasen (Fig.19). Sind die Hohlräume nach dem Prinzip der Varianten E, F, G gestaltet, so bilden sich Hohlräume 12, indem Luft durch die äußeren Hülle 1 von außen einströmt. Durch die Luft im Hohlraum 12 entsteht eine Isolierung zwischen der äußeren 1 und der inneren Hülle 4. Sind die Hohlräume nach dem Prinzip der Varianten H und I gestaltet, so isolieren alleinig die luftgefüllten Hohlräume 11.1 bzw. 4.9.

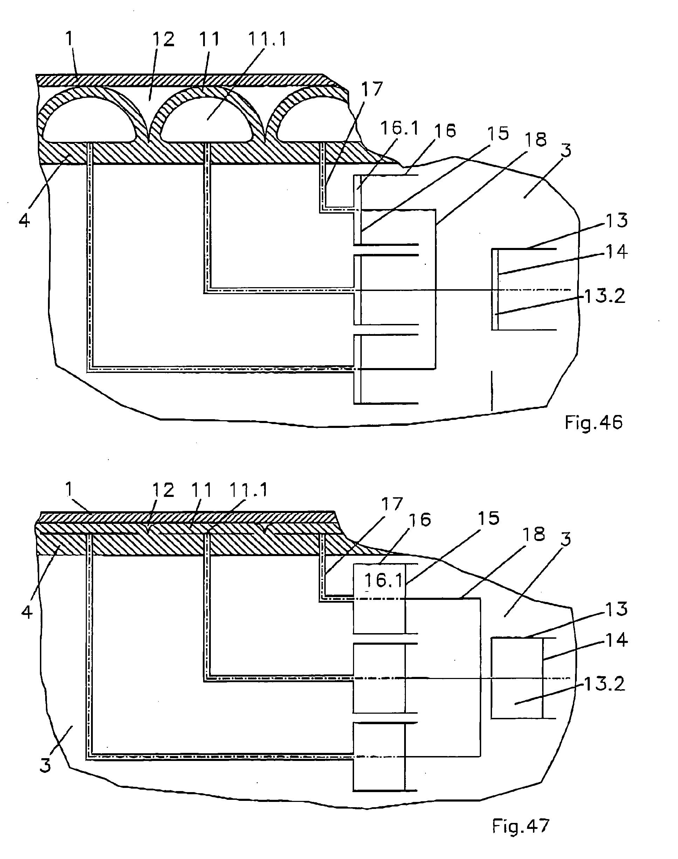

Eine weitere erfindungsgemäße Variante AL der Wärmflasche hat die selben Elemente wie die Variante L. Allerdings handelt es sich hierbei um eine Kühlflasche. Die Variante unterscheidet sich weiterhin durch den Zylinder 14. In ihm befindet sich in dieser Variante das Medium, dessen Siedepunkt zwischen 0° C und 30°C, vorzugsweise zwischen 5° C und 15°C, liegt, im Hohlraum 13.2 auf der Stangenseite des Zylinders 14 (Fig.46). Dieser Hohlraum 13.2 ist, mittels der Stangendichtung des Zylinders 13, nach außen dicht abgeschlossen. Fig.46 zeigt die Kühlflasche in einem Zustand, in dem die Temperatur des Wassers 3 und die des Mediums im Hohlraum 13.2 unter der Siedetemperatur des Mediums liegt und somit das Medium flüssig ist. Erwärmt sich nun die Kühlflasche über die Temperatur des Siedepunktes des Mediums, so verdampft dieses, dehnt sich aus, vergrößert den Hohlraum 13.2, bewegt den Kolben 14 nach rechts (Fig.47) und bewegt somit Ober die Stangen 18 auch die Kolben 15 nach rechts, vergrößert den Hohlraum 16.1, verkleinert somit den Hohlraum 11.1 und verringert somit die Isolierwirkung der Kühlflasche.

Eine weitere erfindungsgemäße Variante M der Wärmflasche funktioniert nach dem gleichen Funktionsprinzip wie die Variante L. Jeder der Zylinder 16 mit Kolben 15 bildet hier, ebenso wie in Variante L, auf der Kolbenseite einen Hohlraum 16.1, der über Leitungen 17 mit je einem der Hohlräume 11.1 bzw. 4.9 verbunden ist (Fig.44). Allerdings gibt es keine Stangen 18 und keinen Zylinder 13 mit Kolben 14 und hier sind die Zylinder 16 auf der anderen Kolbenseite geschlossen und bilden dort einen abgeschlossenen Hohlraum 16.2. Dieser Hohlraum ist wie in der Variante L mit einem Medium gefüllt das bei normaler Raumtemperatur von etwa 20°C flüssig ist und dessen Siedepunkt zwischen 30°C und 100°C, vorzugsweise zwischen 35°C und 80°C, liegt. Fig. 44 zeigt das Medium bei einer Temperatur, die über dem Siedepunkt des Mediums liegt. In Fig. 45 ist das Medium flüssig.

Eine weitere erfindungsgemäße Variante N der Wärmflasche bzw. Kühlflasche funktioniert nach dem gleichen Funktionsprinzip wie die Variante L. Sie besitzt die gleichen Hohlräume 11.1 bzw. 4.9, die über Leitungen 17 mit Zylindern 16 verbunden sind und die mit der Kolbenseite 16.1 des Zylinders 16 einen gemeinsamen Hohlraum bilden, der mit dem Medium Luft, Wasser, Gas oder Öl gefüllt ist. Im Gegensatz zur Variante L gibt es allerdings keinen Zylinder 13 mit Kolben 14. Außerdem können die Kolben 15 vom Bediener von Hand bewegt werden. Dazu ragen die Kolbenstangen entweder aus der Wärmflache heraus, oder die Zylinder 16 mit Kolben 15 sind gänzlich außerhalb der Wärmflasche angebracht, oder die Kolben 15 sind über Stellglieder bewegbar, welche für den Bediener zugänglich sind. Vorteilhafterweise sind die Stellglieder miteinander verbunden, damit durch das Bewegen von einem Stellglied gleichzeitig alle Kolben 15 bewegt werden. Schiebt der Bediener die Kolben 15 in den Zylinder 16, so dass sich der Hohlraum 16.1 verkleinert, so wird das Medium aus den Zylindern 16 in die Hohlräume 11.1 bzw. 4.9 verdrängt, welche sich folglich vergrößern und somit die Isolierwirkung vergrößern. Dieses manuelle Einstellen der Isolierung ist unabhängig davon, ob es sich um eine Kühlflasche oder um eine Wärmflasche handelt.
Alternativ zum manuellen Verschieben der Kolben 15 werden diese automatisch über einen Temperaturregler bewegt. Dieser funktioniert wie ein Thermostat eines Heizkörpers. Es ist vom Wasser 3 der Wärmflasche umgeben und arbeitet mit ein Bimetall das sich bei Temperaturänderung verformt. Ändert sich die Temperatur des Wassers 3, so bewegt das Thermostat die Kolben 15 in den Zylindern 16 und ändert somit das Volumen der Hohlräume 11.1 bzw. 4.9 und damit auch die Isolierwirkung. Das Bimetall im Thermostat ist dabei so angeordnet, dass es bei der Kühlflasche mit zunehmender Erwärmung des Wassers 3 die Isolierung verringert. Bei der Wärmflasche hingegen ist es so angeordnet, dass es bei zunehmender Abkühlung des Wassers 3 die Isolierung verringert.

Eine weitere erfindungsgemäße Variante Y der Wärmflasche funktioniert nach dem gleichen Funktionsprinzipen wie die Varianten M und N. Statt mehreren Hohlräumen 11.1 bzw. 4.9, verfügt diese Variante allerdings nur über einen Hohlraum 4.9 wie in Variante Z. Dieser Hohlraum ist über eine Leitung 17 mit einen Zylinder 16 verbunden. Außerdem wird in Variante Y, bei gleichen Funktionsprinzip wie Variante N, für den einen Zylinder nur ein Stellglied benötigt.

Eine weitere erfindungsgemäße Variante O der Wärmflasche funktioniert nach dem gleichen Funktionsprinzip wie die Varianten E, F, G, K, L, M, oder N, jedoch verzichtet sie auf eine äußere Hülle 1, oder wie Variante J auf eine zweite äußere Hülle 1.1. Dies ergibt gegenüber den anderen Varianten mit einer äußeren Hülle eine Kostenersparnis. Die Isolierung ist dadurch insgesamt geringer als bei den anderen Varianten.

Die folgenden erfindungsgemäßen Varianten P und Q der Wärmflasche besitzen eine innere Hülle 4 und eine äußere Hülle 1. Die äußere Hülle 1 ist luftdurchlässig oder hat Löcher. Die innere Hülle 4 ist wasserdicht und bildet im Gebrauchszustand (wenn der Verschlussdeckel der inneren Hülle geschlossen ist) einen geschlossen Volumenkörper. Die innere Hülle 4 wird mit heißem Wasser 3 (etwa 60-100°C) gefüllt und der Verschlussdeckel verschlossen. In diesem Zustand ist der Hohlraum 12, zwischen innerer Hülle 4 und äußerer Hülle 1 (Fig. 21) am größten. Kühlt sich das Wasser 3 auf etwa 30°C ab, so verkleinert sich das Wasservolumen. Die Volumenverkleinerung verformt die innere Hülle 4 derart, dass sich der Hohlraum 12 zwischen innerer Hülle 4 und äußerer Hülle 1 verkleinert (Fig. 22). Dabei fließt Luft aus dem Hohlraum 12, durch die äußere Hülle 1, nach außen ab. Die Folge ist eine verminderte Isolationswirkung des Hohlraums 12.
Bei der erfindungsgemäßen Variante P der Wärmflasche besitzt die innere Hülle 4 einen Boden 4.2 und mehrere Reservoire 4.3, die eine Öffnung 4.8 zum Wasser 3 haben. Nach der Befüllung der inneren Hülle 4 mit heißem Wasser 3 (60°C), ist das Wasservolumen in der innere Hülle 4 am größten und das Wasser 3 füllt die Reservoire 4.3 komplett aus, richtet diese auf und dadurch ist der Hohlraum 12, zwischen innerer Hülle 4 und äußerer Hülle 1, in diesem Zustand am größten (Fig. 21). Kühlt sich das Wasser 3 auf etwa 30°C ab, so verkleinert sich das Wasservolumen und zieht die Reservoire 4.3 derart zusammen, dass sich der Hohlraum 12 zwischen innerer Hülle 4 und äußerer Hülle 1 verkleinert (Fig. 22).
Bei der erfindungsgemäße Variante Q der Wärmflasche besitzt die innere Hülle 4 tellerförmige Elemente 4.5 auf denen Abstandhalter 4.4 sitzen. Ist das Wasser heiß (etwa 60°C), so füllt das Wasservolumen die innere Hülle 4 derart aus, dass sich die tellerförmigen Elemente 4.5 verformen, somit die Abstandshalter 4.4 die äußere Hülle 1 von der inneren Hülle 4 wegdrücken und den Hohlraum 12, zwischen innerer Hülle 4 und äußerer Hülle 1, vergrößern (Fig. 23). Kühlt sich das Wasser 3 auf etwa 30°C ab, so verkleinert sich das Wasservolumen und zieht die tellerförmigen Elemente 4.5 mit den Abstandhaltern 4.4 nach innen, so dass sich der Hohlraum 12 zwischen innerer Hülle 4 und äußerer Hülle 1 verkleinert (Fig. 24). Damit sich bei einer Volumenänderung des Wassers nicht die gesamte innere Hülle 4, sondern nur die tellerförmigen Elemente 4.5 verformen, müssen die tellerförmigen Elemente elastischer sein, als der Rest der inneren Hülle 4. Dieser Rest der inneren Hülle 4 wird deshalb, beispielsweise durch Seile 4.6, -die den ganzen Wärmflaschenumfang umspannen (gestrichelt dargestellt), versteift. Diese Seile bestehen aus Material das steifer ist als das der inneren Hülle. Alternativ wird die innere Hülle 4 an den Stellen, außerhalb der tellerförmigen Elementen, durch Verstärkungen 4.7 (gestrichelt dargestellt) versteift.
Die erfindungsgemäße Variante R der Wärmflasche entspricht der Variante Q, allerdings sind die Elemente der inneren Hülle 4 die sich bei einer Volumenänderung des Wassers verformen nicht tellerförmig, sondern es sind längliche V-förmige Elemente 4.5 (Fig. 23+24) oder Ringe. Sind es Ringe, so erstrecken sich diese über den gesamten Umfang der Wärmflasche. Die Abstandhalter 4.4 und die Verstärkungen 4.7 (gestrichelt dargestellt) sind ebenfalls längliche Elemente oder Ringe, die sich über den Umfang der Wärmflasche erstrecken.

Die erfindungsgemäße Variante S einer Kühlflasche funktioniert auch nach dem Prinzip (wie Variante Q), dass die Volumenänderung des Wassers 3 in der inneren Hülle 4, die innere Hülle 4 verformt, allerdings funktioniert sie als Kühlflasche, also umgekehrt wie eine Wärmflasche. Das Wasser 3 wird bei Raumtemperatur in die Kühlflasche eingefüllt und diese wird verschlossen, dabei legt sich die innere Hülle 4 an die äußere Hülle 1 an (Fig.48). Nun wird die Kühlflasche im Kühlschrank abgekühlt, dabei verringert sich das Volumen der Wasserfüllung 3, so dass sich die innere Hülle 4 in den Zustand von lauter aneinander gereihten V-Pofilen verformt (Fig.49). Der vergrößerte Hohlraum 12 erhöht die Isolationswirkung.

Die erfindungsgemäße Variante T einer Kühlflasche funktioniert nach dem gleichen Prinzip wie die Variante S, allerdings ist in dieser Variante die äußere Hülle 1 dehnbarer als in der Variante S. Erwärmt sich die Wasserfüllung und vergrößert sich somit das Volumen des Wassers 3, so bewirkt dies eine Streckung der inneren Hülle 4 (Fig.50), bei gleichzeitiger Dehnung der äußeren Hülle 1. Als Folge legt sich die innere Hülle 4 an die äußere Hülle 1 an und der verkleinerte Hohlraum 12 verringert die Isolationswirkung. Wird hingegen die verschlossene Kühlflasche und somit auch das Wasser 3 wieder im Kühlschrank abgekühlt, so verringert sich das Volumen des Wassers 3 und daraufhin verformt sich die innere Hülle 4 und vergrößert somit den Hohlraum 12 (Fig.51).

Bei der erfindungsgemäße Variante U der Wärmflasche besitzt die innere Hülle 4 einen Boden 4.2 und mehrere Reservoire 4.3, die eine Öffnung 4.8 zum Wasser 3 haben. Die innere Hülle 4 und die äußere Hülle 1 sind über Abstandshalter 20 verbunden. Zuerst wird heißes Wasser 3 (etwa 60°C), in die innere Hülle 4 der Wärmflasche gefüllt und diese mit einem Verschluss verschlossen. In diesem Zustand sind die Reservoire 4.3 vollständig gefüllt und samt den Abstandshaltern aufgerichtet (Fig.29). Kühlt sich das Wasser 3 ab, so verkleinert es sein Volumen, zieht sich aus den Reservoiren 4.3 zurück und faltet diese somit zusammen. Durch das Zusammenfaltern der Reservoire 4.3, bewegt sich die äußere Hülle 1 nach rechts und klappt dadurch die Abstandshalter nach rechts (Fig.30). In Folge dessen verkleinert sich der Hohlraum 12 und verringert somit dessen Isolationswirkung.

Die erfindungsgemäße Variante X erwärmt oder kühlt die Wärm- oder Kühlflasche mittels elektrischen Elementen. Handelt es sich um eine Wärmflasche, so funktioniert sie wie die Varianten E, F, F1, F2, H, I, L, M, N, P, Q, V, U, Y, Z, jedoch wird das Wasser 3 hierbei durch eine Heizspirale erwärmt, die sich innerhalb der Wärmflasche direkt im Wasser 3 befindet, oder durch Heizdraht der in die innere Hülle 4 eingewebt ist und somit auch Kontakt zum Wasser 3 hat. Zum Erwärmen wird die Heizspirale, oder der Heizdraht an eine Stromversorgung angeschlossen. Hat das Wasser die gewünschte Temperatur erreicht, dann kann die Stormversorgung unterbrochen werden und man ist unabhängig von dieser und es kann in der Wärmflasche kein Kurzschluss und kein Elektrosmog entstehen.
Handelt es sich um eine Kühlflasche so funktioniert sie wie die Varianten S oder T, allerdings befindet sich innerhalb der Kühlflasche ein elektrisches Kühlelement, welches das Wasser 3 in der inneren Hülle 4 abkühlt.

Bei der erfindungsgemäßen Variante V einer Wärmflasche ist der Raum zwischen innerer Hülle 4 und äußerer Hülle 1 mit einem Gel 21 gefüllt (Fig.37 und 38). Das Gel 21 enthält das gleiche Medium wie in Variante E, welches bei Raumtemperatur von etwa 20°C flüssig ist und bei einer Temperatur die höher liegt als die Siedetemperatur des Mediums (vorzugsweise zwischen 40° C und 80°C) gasförmig wird. Das Medium befindet sich zwischen dem Gel 21 und der äußeren Hülle 1 (82), oder es befindet sich im inneren des Gels 21 (B1). Im Fall B1 wird bei der Herstellung der Wärmflasche das Medium in das Gel 21 injiziert, oder mechanisch untergemischt. Im Fall B2 wird bei der Herstellung der Wärmflasche das Medium über Pipetten auf die Geloberfläche getropft, oder es mittels Sprühdüsen aufgesprüht.
Wird heißes Wasser 3 mit einer Temperatur, die höher als der Siedepunkt des Mediums 22 ist, in die innere Hülle 4 der Wärmflasche gefüllt, so erhitzt sich auch das Gel 21 und das Medium 22 verdampft und vergrößert somit sein Volumen und somit auch die Isolationswirkung der Wärmflasche (Fig.38). Dabei ist die äußere Hülle 1 so elastisch, dass sie sich an die Volumenvergrößerung des Mediums 22 anpassen kann. Kühlt sich das Wasser 3 ab, so kühlt sich auch das Medium 22 ab. Fällt die Temperatur des Mediums 22 unter seinen Siedepunkt, so wird dies wieder flüssig, verkleinert sein Volumen und verringert somit die Isolationswirkung der Wärmflasche (Fig.37). Alternativ befindet sich in der inneren Hülle statt Wasser 3 Gel. In dieser Ausführung wird die Wärmflasche in der Mikrowelle erhitzt. Bei einer anderen Alternative wird auf die innere Hülle 4 und die Wasserfüllung 3 verzichtet. In diesem Fall besteht die Füllung der äußeren Hülle 1 gänzlich aus Gel 21 und dem Medium 22. Bei dieser Ausführung wird die Wärmflasche in der Mikrowelle erhitzt.

Bei der erfindungsgemäßen Variante W ist der Raum zwischen der inneren Hülle 4 und der zweiten inneren Hülle 6, wie in Variante V, mit einem Gel 21 und einem Medium 22 gefüllt, welches bei Raumtemperatur von etwa 20°C flüssig ist und bei einer Temperatur die höher liegt als der Siedepunkt des Mediums (vorzugsweise zwischen 40° C und 80°C) gasförmig wird. Wird heißes Wasser 3 mit einer Temperatur, die höher als der Siedepunkt des Mediums 22 ist, in die zweite innere Hülle 6 der Wärmflasche gefüllt, so erhitzt sich auch das Gel 21 und das Medium 22 verdampft und vergrößert somit sein Volumen. Diese Volumenvergrößerung drückt, wie in Variante Q, die Abstandshalter 4.4 nach außen und vergrößert dadurch den Hohlraum 12, zwischen innerer Hülle 4 und äußerer Hülle 1 (Fig. 39). Kühlt sich das Wasser 3 auf eine Temperatur unter dem Siedepunkt des Mediums ab (etwa 30°C), so kühlt auch das Medium 22 ab, kondensiert, verkleinert sein Volumen und zieht somit die tellerförmigen Elemente 4.5 mit den Abstandhaltern 4.4 nach innen, so dass sich der Hohlraum 12 zwischen innerer Hülle 4 und äußerer Hülle 1 verkleinert (Fig. 40).
Alternativ wird auf die zweite innere Hülle 6 und die Wasserfüllung 3 verzichtet. In diesem Fall besteht die Füllung der inneren Hülle 4 gänzlich aus Gel 21 und dem Medium 22. Bei dieser Alternative wird die Wärmflasche in der Mikrowelle erhitzt.

Die erfindungsgemäße Variante W 1 ist wie die Varianten P, Q, S, T, oder U aufgebaut. Allerdings befindet sich in dieser Variante statt Wasser 3, Gel in der inneren Hülle 4. In dem Gel befindet sich, wie im Gel 21 der Variante V, ein Medium 22, welches bei Raumtemperatur von etwa 20°C flüssig ist und bei einer Temperatur die höher liegt als der Siedepunkt des Mediums (vorzugsweise zwischen 40° C und 80°C) gasförmig wird. Bei dieser Variante wird die Wärmflasche in der Mikrowelle erhitzt. Steigt so die Temperatur des Mediums 22 über die Siedetemperatur des Mediums 22 an, so verdampft das Medium 22 und vergrößert somit sein Volumen. Diese Volumenvergrößerung vergrößert auf die gleiche Art und Weise, wie die Volumenänderung des Wassers 3 in den Varianten P, Q, S, T, oder U, den Hohlraum 12, zwischen innerer Hülle 4 und äußerer Hülle 1. Kühlt sich das Gel 21 und somit auch das Medium 22 auf eine Temperatur unter dem Siedepunkt des Mediums 22 ab, so kondensiert das Medium 22 und verkleinert somit den Hohlraum 12 zwischen innerer Hülle 4 und äußerer Hülle 1 und die Isolierwirkung des Hohlraums 12 wird verringert.

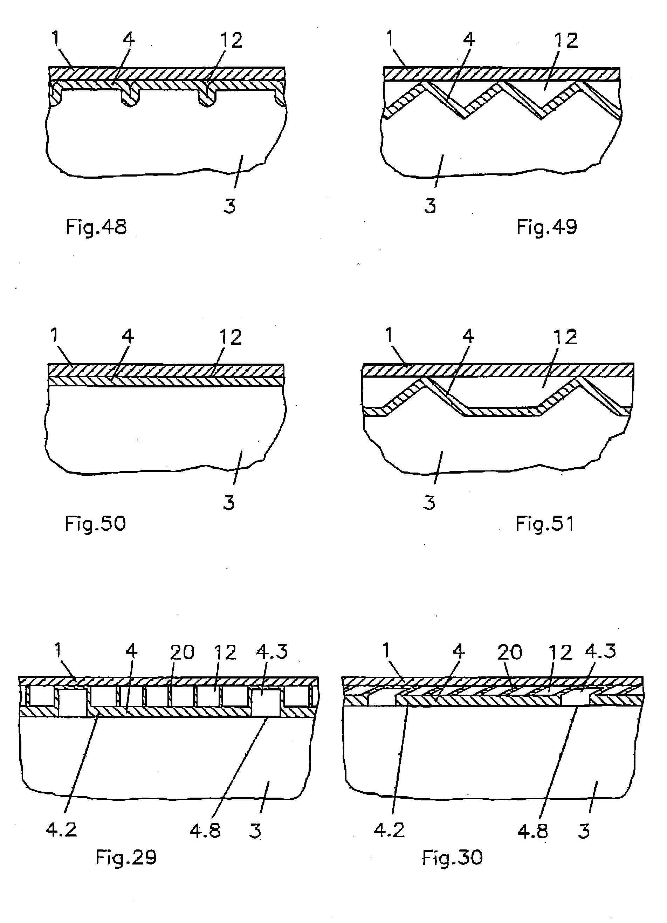

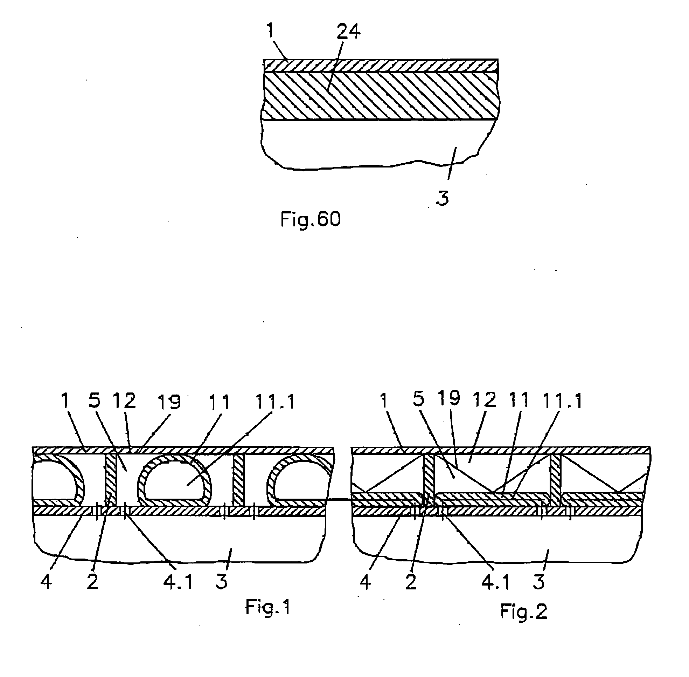

Die erfindungsgemäße Variante XA ist eine Kühlflasche. Zu ihr gehört eine luftdurchlässige äußeren Hülle 1 und eine inneren Hülle 4 mit Löchern 4.1. Die innere Hülle 4 und die äußere Hülle 1 sind mit Trennwänden 2 verbunden, wodurch sich die Hohlräume 5 bilden. Auf die innere Hülle 4 sind geschlossene Hohlkörper 11 geklebt. An jeden Hohlkörper ist eine wasserdichte Folie 19 geklebt, die auch noch zwischen die Verbindung der Trennwände 2 und der äußeren Hülle 1 geklebt ist. Der Hohlraum 11.1 der Hohlkörper 11 ist mit einem Medium gefüllt, dessen Siedepunkt zwischen 0°C und 30°C, vorzugsweise zwischen 5° C und 15°C, liegt.
Zunächst wird die innere Hülle 4 der Kühlflasche mit einer Flüssigkeit, vorzugsweise Wasser 3, gefüllt und verschlossen. Indem das Wasser 3 wärmer ist, als der Siedepunkt des Mediums, wird das Medium im Hohlraum 11.1 ebenfalls erwärmt und wird gasförmig, dehnt sich somit aus und bläht den Hohlkörper 11 zu einer fast kreisrunden Form auf (Fig. 1). Dadurch wird die Folie 19 an die äußere Hülle 1 gedrückt und der Hohlraum 5 vergrößert. Dabei strömt in den Hohlraum 5 Wasser 3 durch die Löcher 4.1 aus der inneren Hülle 4 nach. Anschließend wird die Kühlflasche im Kühlschrank auf eine Temperatur von etwa 3°C abgekühlt. Diese Temperatur liegt unter dem Siedepunkt des Mediums im Hohlraum 11.1. Dadurch wird das Medium flüssig, verkleinert sein Volumen und zieht den Hohlraum 11.1 zusammen (Fig.2). Dadurch wird die Folie 19 von der äußeren Hülle 1 weggezogen und somit der Hohlraum 12 vergrößert, der sich mit Luft von außerhalb der Kühlflasche füllt, welches durch die luftdurchlässige äußere Hülle 1 eindringen kann. Das überschüssige Wasser 3 aus dem Hohlraum 5 fließt durch die Löcher 4.1 zurück ins innere der Kühlflasche. In diesem Zustand ist durch die Luft im Hohlraum 12 die Isolation der Kühlflasche am größten. Erwärmt sich die Kühlflasche auf eine Temperatur, die über dem Siedepunkt des Mediums liegt, so wird dieses gasförmig, dehnt sich somit aus und bläht den Hohlkörper 11 zu einer fast kreisrunden Form auf (Fig. 1). Dadurch wird die Folie 19 an die äußere Hülle 1 gedrückt und der Hohlraum 5 vergrößert und der Hohlraum 12 verkleinert (Fig.1). Dabei strömt in den Hohlraum 5 Wasser 3 durch die Löcher 4.1 aus der inneren Hülle 4 nach und die Luft des Hohlraums 12 entweicht durch die luftdurchlässige äußere Hülle 1. Indem der luftgefüllte Hohlraum 12 somit nicht mehr besteht, hat er auch keine isolierende Wirkung mehr.

Die Beschreibung ist auch Bestandteil des erfinderischen Gedankens.

Nummerierung entsprechend der Zeichnungen:
- 1: äußeren Hülle
- 1.1: zweite äußere Hülle
- 2: Trennwand
- 3: Wasser
- 3.1: Wasserspiegel
- 4: innere Hülle
- 4.1: Loch
- 4.2: Boden
- 4.3: Reservoir
- 4.4: Abstandshalter
- 4.5: Teller/V-Ring
- 4.6: Seil
- 4.7: Verstärkung
- 4.8: Öffnung
- 4.9: Hohlraum
- 4.10: Hohlraum
- 5: Hohlraum
- 6: zweite innere Hülle
- 6.1: Loch
- 7: Richtung Wärmflaschenverschluss
- 8: Richtung: weg von Wärmflaschenverschluss
- 9: Luft
- 10: Hohlraum
- 11: Hohlkörper
- 11.1: Hohlraum
- 12: Hohlraum
- 13: Zylinder
- 13.1: Hohlraum
- 13.2: Hohlraum
- 14: Kolben
- 15: Kolben
- 16: Zylinder
- 16.1: Hohlraum
- 16.2: Hohlraum
- 17: Leitung
- 18: Stange
- 19: Folie
- 20: Abstandshalter
- 21: Gel
- 22: Medium
- 23: Wärmflasche
- 23.1: Rand
- 23.2: Einfüllstutzen
- 24: Schaumstoff

## Patentansprüche

1. Wärmflasche oder Kühlflasche für Mensch oder Tier, **dadurch gekennzeichnet, dass** die Isolierwirkung der Wärmflasche mit zunehmender Abkühlung der Wärmflasche, oder mit zunehmender Erwärmung der Kühlflasche abnimmt, oder die lsollerwirkung manuell verändert werden kann.

2. Wärmflasche nach Anspruch 1, **dadurch gekennzeichnet, dass** wenigstens eine innere Hülle 4 von Hohlräumen 5 umgeben ist und sich innerhalb der inneren Hülle 4 wenigstens eine zweite innere Hülle 6 befindet, in die eine Flüssigkeit, vorzugsweise Wasser, gefüllt wird.

3. Wärmflasche nach Anspruch 1, **dadurch gekennzeichnet, dass** wenigstens ein Hohlraum 11.1 bzw. 4.9 bzw. 4.10, der sich im Randbereich der Wärmflasche befindet, mit wenigstens einem bei 20°C flüssigen Medium, vorzugsweise Alkohol, gefüllt ist, dass bei einer Temperatur zwischen 30°C und 100°C, vorzugsweise zwischen 40°C und 80°C, verdampft und dass die innere Hülle 4 mit einer Flüssigkeit, vorzugsweise Wasser 3, oder mit einem Gel, oder mit Granulat, oder mit Körnern gefüllt ist.

4. Wärmflasche nach Anspruch 1, **dadurch gekennzeichnet, dass** wenigstens ein Hohlraum 11.1, der sich im Randbereich der Wärmflasche befindet, mit einem Reservoir verbunden ist, dass über den Temperaturbereich von 10 bis 100°C ein nahezu konstantes Volumen hat. Das Reservoir mit wenigstens einem Medium, vorzugsweise Wasser, gefüllt ist, dass bei Erwärmung sein Volumen stärker vergrößert, als das Volumen des Reservoirs und dass die innere Hülle 4 mit einer Flüssigkeit, vorzugsweise Wasser 3, oder mit einem Gel, oder mit Granulat, oder mit Körnern gefüllt ist.

5. Wärmflasche nach Anspruch 1, **dadurch gekennzeichnet, dass** die äußeren Hülle 1 mit einem Gel 21 gefüllt ist, indem sich wenigstens ein bei 20°C flüssiges Medium 22, vorzugsweise Alkohol, befindet, dass bei einer Temperatur zwischen 30°C und 100°C, vorzugsweise zwischen 40°C und 80°C, verdampft.

6. Wärm- oder Kühlflasche nach Anspruch 1, **dadurch gekennzeichnet, dass** wenigstens eine flüssigkeitsdurchlässige innere Hülle 4 mit wenigstens einer flüssigkeitsdichten äußeren Hülle 1, wenigstens einen Hohlraum 5 bildet und dass die innere Hülle 4 mit einer Flüssigkeit, vorzugsweise Wasser 3, gefüllt wird.

7. Kühlflasche nach Anspruch 1, **dadurch gekennzeichnet, dass** wenigstens ein Hohlkörper mit einem Medium gefüllt ist, dessen Siedepunkt zwischen 0°C und 30°C, vorzugsweise zwischen 5° C und 15°C, liegt und der eine Folie 19 bewegt, die durch diese Bewegung einen Hohlraum 12 vergrößert oder verkleinert.

8. Wärm- oder Kühlflasche nach Anspruch 1, **dadurch gekennzeichnet, dass** sich in der flüssigkeitsdichten äußeren Hülle 1 ein flüssigkeitsdurchlässiger Schaumstoff 24 befindet und dass der Schaumstoff 24 mit einer Flüssigkeit, vorzugsweise Wasser 3, gefüllt wird.

9. Verfahren zum Wärmen oder Kühlen von für Mensch oder Tier mit einer Wärmflasche oder Kühlflasche, **dadurch gekennzeichnet, dass** die Isolierwirkung der Wärmflasche mit zunehmender Abkühlung der Wärmflasche, oder mit zunehmender Erwärmung der Kühlflasche abnimmt, oder die Isolierwirkung manuell verändert werden kann.

10. Verfahren zum Wärmen mit einer Wärmflasche nach Anspruch 9, **dadurch gekennzeichnet, dass** wenigstens ein Hohlraum 11.1 bzw. 4.9, der sich im Randbereich der Wärmflasche befindet und mit wenigstens einem Zylinder 16 einen abgeschlossenen Hohlraum 16.1 bildet und der Kolben 15 des Zylinders 16 die Füllung dieser Hohlräume aus, vorzugsweise Luft, oder Gas, oder Öl, oder Wasser, zwischen den Hohlräumen 11.1 bzw. 4.9 und 16.1 verändert und der Kolben 15 des Zylinders 16 manuell, oder über ein Thermostat, oder über die Volumenänderung eines Mediums bewegt wird und das Medium, vorzugsweise Alkohol ist und bei 20°C flüssig ist und dass das Medium bei einer Temperatur zwischen 30°C und 100°C, vorzugsweise zwischen 35°C und 80°C, verdampft und dass das Medium oder das Thermostat von der Füllung der inneren Hülle 4 erwärmt wird und dass die Füllung der inneren Hülle 4 aus Gel, oder Körnern, oder Granulat, oder Flüssigkeit, vorzugsweise Wasser 3, besteht.
